Europäisches Patentamt

European Patent Office

Office européen des brevets

(19)

(11) **EP 0 519 336 B1**

(12) **EUROPEAN PATENT SPECIFICATION**

(45) Date of publication and mention
of the grant of the patent:
**15.10.1997 Bulletin 1997/42**

(51) Int Cl.$^6$: **C12N 15/67**, C12N 15/31,
C12N 5/10, C12N 15/52,
C12Q 1/02, C12Q 1/68

(21) Application number: **92109881.0**

(22) Date of filing: **12.06.1992**

(54) **Chimeric gene comprising a promoter-regulater and a gene coding for an indicator protein**

Promotor-Regulator und das für Indikator-Protein kodierende Gen enthaltendes chimäres Gen

Géne chimérique comprenant un promoteur-régulateur et un gène codant pour une protéine indicatrice

(84) Designated Contracting States:
**AT BE CH DE DK ES FR GB GR IT LI LU NL PT SE**

(30) Priority: **18.06.1991 GB 9113097**

(43) Date of publication of application:
**23.12.1992 Bulletin 1992/52**

(73) Proprietor: **BOEHRINGER INGELHEIM INTERNATIONAL GmbH**
**55218 Ingelheim am Rhein (DE)**

(72) Inventors:
• **Taniguchi, Tadatsugu, Dr.**
**Osaka 567 (JP)**
• **Barsoumian, Edward L., Dr.**
**Mino, Osaka Pref. 562 (JP)**
• **Shibuya, Hiroshi, Dr.**
**Sanda-shi, Hyogo 669-13 (JP)**
• **Swetly, Peter, Prof. Dr.**
**A-1130 Vienna (AT)**

(74) Representative: **Laudien, Dieter, Dr. et al**
**Boehringer Ingelheim GmbH**
**Abteilung Patente**
**55216 Ingelheim (DE)**

(56) References cited:
WO-A-89/05864        WO-A-90/10710
WO-A-91/01379        WO-A-92/14839

• **ANAL. BIOCHEM., 176(1), pp 28-32, 1989; WILLIAMS, THOMAS M. ET AL: 'Advantages of firefly luciferase as a reporter gene: application to the interleukin -2 gene promoter'**
• **MOLECULAR AND CELLULAR BIOLOGY, April 1987, WASHINGTON US, pages 1576-1579; F. MAXWELL ET AL: 'Cloning, sequence determination, and expression in transfected cells of the coding sequence for the tox 176 attenuated Diphteria toxin A gene'**

## Description

<u>Introduction</u>

The immune response is essential for the defense of the organism and plays a major role in the rejection of organ transplants and in certain aspects of autoimmunity. Therefore, there is an ongoing search for natural or synthetic products to regulate or to control the immune responses. One of the initial and central events in mounting an immune response is to bring about activation of specific T cells by means of antigens. Following the initial activation, clonal expansion of T cells is achieved through the generation of an autocrine ligand, such as IL-2 and expression of its receptor IL-2R($\alpha$,$\beta$). This allows an accelerated expansion of the T cell population needed for defense of the host against microorganisms (bacteria, virus, etc.) or for cell mediated cytotoxicity against aberrant cells (cancer cells) as well as the T cell population raised against cells expressing foreign surface antigens (Organ transplants) (1-4).

<u>Activation of T cells:</u>

T cell activation is central in the initiation and regulation of immune responses. In the past decade, functional analysis of T cells and their surface components have identified and characterized receptors and other membrane proteins which participate in the transduction of signals through specific ligand mediators such as IL-2, IL-4 etc. (5-9).

Antigen presented by an antigen-presenting cell (APC) to the T cell receptor leads to membrane phospholipid turnover, characterized particularly by the hydrolysis of phosphatidylinositol 4,5 biphosphate ($PIP_2$), generation of diacylglycerol and inositol 1,4,5-triphosphate, mobilization of intracellular calcium ions, and phosphorylation of a number of cellular proteins. These biochemical events involve the $T_1$ - $T_3$ complex (10, 11). It is thought that a second signal is provided by the APC (in this instance the macrophage). Surface mediated action can be mimicked by phorbol esters, which bypass the membrane events, but involve the calcium-mediated event leading to the activation of protein kinase C (PKC). It is however, unclear how the cell membrane and cytoplasmic events lead to the coordinated expression of specific groups of genes in a sequential manner (12).

<u>Cytokines</u>

The antigen-mediated induction leads to the activation of a number of T cell genes, also known as cytokine genes. Cytokines also known as lymphokines (lymphocyte-derived) exert very important biological effects on T cells, B cells and other tissues, through autocrine or paracrine processes leading to biological events such as cellular expansion, differentiation or maturation (13-16).

<u>Interleukin 2</u>

For T-cell mediated immune responses, a cytokine, in the form of interleukin 2 (IL-2) is essential and critical. IL-2 is the first known cytokine generated by activated T cells; it is transiently produced, and acts on the target cells in an autocrine or paracrine manner leading to their clonal expansion. IL-2 acts on T cells by interacting with a receptor complex composed of two membrane peptide components, the IL-2 R$\alpha$ (55Kd) and the IL-2R$\beta$ (75 Kd) molecules (17-19).

The mechanism by which the IL-2-mediated signal is carried out remains obscure, although it is thought to involve protein kinase activity (20).

<u>The human IL-2 gene regulatory region</u>

As indicated by a number of studies, the generation of IL-2 is strictly controlled and regulated, moreover, only the thymus-derived mature T cells are the source of IL-2 which is generated following antigenic or mitogenic stimulation. Early studies have demonstrated regulation of IL-2 production at the transcriptional level (21-24), although some Posttranscriptional regulatory mechanism(s) have also been suggested. The cloning and the genomic characterization of human and murine IL-2 genes has revealed the presence of well conserved DNA sequences within the 5' flanking region (25-26).

Williams *et al. (Anal. Biochem.* 176:28-32 (1989)) teach a genetic construct wherein a part (587 bp) of the promoter regulator region of the IL-2 gene is combined with the luciferase gene as reporter. This arrangement is reported to provide are more sensitive assay to study the IL-2 promoter as compared to the previously known CAT assay.

The functional DNA sequences important in the transcriptional regulation of IL-2 gene expression were characterized by excising the putative regulatory DNA sequences from human IL-2 chromosomal gene and fusing them to the chloramphenicol acetyltransferase (CAT) structural gene. Using such transient expression systems, the functional properties of DNA sequences were elucidated (27). The functional boundary was located between -319 and -264 from the cap or transcription start site. This was further confirmed by $S_1$ mapping analysis of the transcript in induced cells (27).

Detailed Characterization

Analysis of the 5' as well as the 3' deletion mutants revealed that the minimal length of DNA required for controlled IL-2 gene expression is between 192 bp (-319 to -127) and 119 bp (-264 to -145). Furthermore, the segment between -81 and -127 contributed to the maximal activation of the IL-2 gene 5' flanking region, since deletion of this segment showed a reduction in the expression of the indicator CAT gene (27).

Moreover, a functional study of this DNA segment (-319 to -127) linked to heterologous promoter sequences such as that of human IFNβ and SV40 demonstrated that this region alone can confer the specificity of IL-2 gene expression, and it can function in orientation-independent manner. These findings demonstrated or identified the DNA segment containing the regulatory enhancer, whose function is restricted to activated T cells (27).

It is important to note that the identified regulatory DNA sequences contain purine-rich regions (Pu-boxes), and these are common or homologous to other T cell specific genes such as other cytokine genes (28-30).

Detailed characterization of the 5' flanking regulatory region of the human IL-2 gene revealed the presence of several functional regions extending from -52 to -98, -127 to -151, and -222 to -319 for the induced transcription of the gene in the human leukemic cell line, Jurkat and in the mouse thymoma cell line, EL-4 (31).

Analysis using 3' deletion mutants revealed further the importance of that region in controlling the inducible activity of IL-2 gene. In fact, a 3' deletion extending to -98 showed a dramatic loss of inducible activity. Moreover, this inducible activity was completely abrogated when the deletion was extended to -145 (31-33).

The detailed analysis revealed DNA-factor binding sites correspond to Pu-boxes (27-33).

In addition to the positive regulatory factors regulating the inducible expression of human IL-2 gene, the presence of a negative regulatory element has been suggested (34). Durand and colleagues reported five nuclear factors which interact with the identified regulatory elements of IL-2 gene (32).

Other studies reported ubiquitous TPA-activated factors, AP-1 and AP-3 interacting with TPA-responsive elements. The upstream promoter site (UPS) -64 to -94 was reported to be a potential binding site for AP-1, AP-3 and Oct-1 (31, 35). It is suggested that non-ubiquitous factors might be binding to this site, since transfection of UPS in non-T cells abrogates the activity of this DNA element (remains inactive) (29).

The multiplicity of regulatory DNA motifs in the 5'flanking region of human IL-2 gene and the variety of ubiquitous and non-ubiquitous (tissue-specific) factors is indicative of the complexity of the IL-2 gene regulation (36).

Immunosuppression

The role of IL-2 is central for the immune response. IL-2 amplifies the antigen-mediated activation signal of specific T cells. Therefore, the activation stage of T cells is a critical event in the process of immune response. It is possible to target the T cell activation stage by interfering at the level of regulated expression of IL-2.

In the search for potent natural or synthetic products acting specifically on the immune response, a number of potent immunosuppressor drugs have been discovered (37-41). Through the detailed biological and pharmacological characterization of these drugs, we have developed a better understanding of the complexicity of T cell activation. Moreover, these drugs have allowed the discovery of cytoplasmic proteins potentially important in the activation process either directly or by acting on as yet unkown cytoplasmic factors important for the transcription of activation genes (42).

Cyclosporine-A and FK506 are microbial products acting on events distal from the cell-membrane and close to nuclear processes (21). Initial studies on the mechanism of these drugs had targeted IL-2-mediated phenomena. These drugs show a dramatic effect on IL-2 production. Moreover, this is well reflected by the inhibition of IL-2 specific mRNA accumulation (43). In line with these observations Kroenke et al. followed the kinetics of IL-2 mRNA, which is detectable in 3 hours and peaks at 6 hours; they were able to inhibit in a dose-dependent manner lectin induced IL-2 mRNA in human leukemic cells (21-24). In contrast, other marker gene products, HT-3 and HLA-B were not affected (24).

Some aspects of the mechanism of action of Cyclosporine-A and FK506 have been elucidated by the characterization of cytoplasmic proteins, which readily interact with these drugs (42). These proteins have been identified as cyclophilins and FKBP respectively (42, 44, 46). They are ubiquitous in their distribution and constitute between 0.1 and 0.4% of total cytostolic protein (42, 44-46).

Furthermore these proteins catalyze the cis-trans isomerization of proline imidic peptide bonds in oligopeptides (42, 47). Such catalytic activity is important, since it accelerates the folding or refolding of many proteins needed during protein synthesis. However, it is still unclear how the immunosuppressors Cyclosporine A and FK506 interfere in the nuclear events leading to the inhibition of IL-2 and other cytokine production (24, 42).

Recent studies suggest that the above mentioned drugs mediate their immunosuppressive effects by impairing the enhancer activities of the IL-2 gene regulatory region. In other words, these compounds inhibit the function of transacting factors that bind to enhancer elements of the IL-2 gene and presumably to the enhancer of other cytokine genes as well (24, 32, 36).

The regulatory region of IL-2 gene is very important, since it controls the expression of IL-2. Therefore, intervention at the level of T cell activation is very desirable, since it will provide means to regulate or suppress the magnitude of the immune response.

The human IL-2 genomic gene has been characterized by many groups and its regulatory region is well defined (26-27, 31, 32). Moreover, the murine IL-2 gene has also been cloned and its regulatory region shows extensive homology with that of the human (see Figure I) (about 85% at the DNA level) (26, 48).

It is desirable to find a rapid and economic method of screening for compounds which are capable of affecting the regulated expression of bioactive compounds such as cytokines, e.g. IL-2, IL-4, IL-6 etc.

We surprisingly found that the ability of a substance to down-regulate or up-regulate the expression of IL-2 can be determined by observing the expression of an indicator protein. When a chimeric gene containing the structural gene for the indicator protein under the control of the promoter regulator sequence of an IL-2 gene was transfected into a cell containing the complement of cellular functions necessary for control of the chimeric gene expression and subjected to inducing conditions in the presence of such a substance the amount of indicator protein produced was found to relate to the ability of the substance to regulate the expression of IL-2.

According to one aspect of the invention therefore we provide a method for determining whether a substance is capable of affecting the regulation of expression of a bioactive compound, e.g. a cytokine such as IL-2 wherein a cell containing a chimeric gene in which the structural gene of an indicator protein is under the control of the promoter regulator sequence of the gene of the bioactive protein or a functional portion thereof is subjected in the presence of the said substance to a condition which, in the absence of the substance, is capable of inducing or suppressing expression of said chimeric gene, and the production of said indicator protein is observed.

The method is expected to be of most interest in screening for substances capable of suppressing the expression of IL-2 or other cytokines. Thus the chimeric gene will be subjected to a condition capable of inducing expression of said gene (e.g. the presence of TPA) and the affect of the test substance on expression of the protein observed. Thus for example, if the resulting expression is lower than that observed for a control in which the chimeric gene is induced in the absence of the test substance this will be an indication that the test substance is potentially capable of suppressing IL-2 generation in normal cells.

In the preferred form of the method the indicator protein is diphtheria toxin or diphtheria toxin A. The expression of the protein is manifested by cell death which can be easily followed by e.g. determining the reduction of [3H]-thymidine uptake in the cell population, or by microscope observation, determining the presence of cytoplasmic indicator enzymes such as lactic dehydrogenase (LDH) in the supernatants, or by cell staining following fixation.

Another aspect of the invention provides a chimeric gene in which the structural gene of an indicator protein is under the control of the promoter regulatory sequence of a bioactive protein, e.g. a mammalian cytokine gene, e.g. a IL-2 gene, or a functional portion of the promoter regulatory sequence.

Preferably the indicator protein is diphtheria toxin or diphtheria toxin A.

Preferably the promoter regulator sequence is that of human or mouse IL-2 gene.

Yet a further aspect of the invention comprises an expression vector containing a chimeric gene as defined above.

The present invention also comprehends a microbial or mammalian cell transformed by a chimeric gene as aforesaid. For use in the method of the invention a cell will preferably be chosen which contains the complement of cellular regulating factors necessary for control of the chimeric gene expression.

As described below the chimeric gene can be prepared by well known standard methods. The DNA sequences coding for the bioactive compound, e.g. cytokine, regulatory sequences and the indicator protein can be obtained by synthesising such sequences on the basis of published sequence information or isolating such sequences from appropriate known cell lines according to known methods.

The construction of the chimeric gene as well as its incorporation in an expression vector can be carried out according to well-known genetic recombination techniques.

In order to transfect host cells with vectors containing the chimeric gene any of the known standard techniques may be used, for example treatment with calcium phosphate (F.L. Graham and A.J. Van der Eb, Virology, 52, 546 (1978)) or, as in the example described below by electroporation (Doi et al., EMBO J., 8:1953 (1989)).

The method of the invention can potentially enable detection of the inhibitory activity of synthetic compounds or natural substances that affect the regulatory events of gene expression of bioactive compounds such as cytokines, e. g. IL-2, IL-4, IL-6 etc.

Diphtheria toxin an indicator gene product

Highly toxic molecules produced by plants and bacteria can kill mammalian cells. In the case of diphtheria toxin, one such molecule is sufficient to achieve cell killing (49).

Diphtheria toxin (DT) is a 58kd protein made up of two fragments, A and B. Fragment A, the N-terminal 21kd protein and fragment B, the 37kd C-terminal make up the toxic molecule. Fragment B allows the binding of the toxin to a surface receptor and translocation of fragment A through the cell membrane into the cytoplasm (50).

Fragment A, also defined as DT-A is the toxic component. It catalyzes the transfer of the adenosine diphosphate ribose moiety of $NAD^+$ onto a specific site on elongation factor 2 (EF-2), thus blocking or inactivating this important cytoplasmic factor needed in the process of translation (protein synthesis). In other words, DT-A can completely inhibit protein synthesis, leading to cell death (51, 52). It is important to note that DT-A cDNA has been cloned (51, 52).

DNA coding for DT-A can be introduced into cells in plasmid constructs containing tissue-specific cis-acting transcriptional regulatory elements, with the intention of achieving lethal expression in cells containing the complementary factors to which these cells have the DNA elements to respond to. The principle of introducing DT-A by means of a plasmid construct has already been tested by Maxwell and colleagues. These investigators have used a truncated metallothionein promoter coupled to DT-A coding region and transfected with a plasmid containing the chloramphenicol acetyl transferase gene (CAT). Using such transfectants, they have been able to measure DT-A effect on CAT activity. Furthermore, by placing the immunoglobulin heavy chain gene enhancer downstream, they were able to express the reporter gene in a tissue-specific manner such as B cells (53, 54). These studies indicate that the expression of a toxin gene can be regulated in a cell-specific manner by linkage with a defined enhancer.

The coexpression experiments have shown significant inhibition in the expression of CAT activity, whereas the frame-shift mutants have not shown such inhibition. Moreover, transfection of DT-A plasmid in the human embryonic kidney cell line, 293 with neomycin-resistance gene have resulted in no transfectants compared to the controls. The failure to obtain DT-A transfectants has been attributed to the lethal level of expression in 293 cells (54).

It is important to note that an attenuated DT-A (tox 176) has also been characterized molecularly, containing a G to A transition at position 383 of the mature coding sequence. Tox 176 seems to have 1-2 order of magnitude lower toxic activity compared to the control wild type (54, 55).

The regulatory sequences of both human and murine IL-2 gene have been well characterized (25-31, 48). Furthermore, the coding sequence of diphtheria toxin fragment A has also been characterized and expressed using specific promoters and tissue specific enhancers.

In the following example an expression plasmid containing the promoter region of mouse IL-2 coupled to the coding region of DT-A and the cotransfection thereof into mouse thymoma EL-4 cells with the neo-resistance gene is described. Neomycin-resistant transfectants were selected and tested for DT-A lethal expression following a mitogenic stimulus.

Chimeric gene construct

The diphtheria toxin fragment A cDNA (DT-A) was cleaved at the restriction site HhaI in the 5' end of the molecule, and cleaved at the 3'end with Sau3AI followed by filling in at the Sau3AI site.

The 5'-flanking DNA sequence fragment (about 3Kbp long) of mouse IL-2 gene was prepared by cleaving the EcoRI-PstI sites from the M-IL-2-G 70 plasmid vector (21).

The above described two fragments, DT-A and the 5'-flanking region of IL-2 gene were inserted into EcoRI and filled-in XbaI sites of πH3M vector (56) together with a synthetic DNA containing Pst I-HhaI ends and the initiation codon of mouse IL-2 gene as shown below (Figures II and III).

Transfection of EL-4 mouse thymoma cells

The large scale preparation of the expression vector containing the chimeric gene construct (IL-2 DT #8) was made following standard techniques (57).

IL-2 DT #8 chimeric gene construct containing the expression vector πH3M was linearized using EcoRI restriction enzyme, since only one EcoRI site exists.

| DNA | 100 μl (100 μg) |
|---|---|
| Y-50 | 50 μl |
| $H_2O$ | 325 μl |
| EcoRI (enzyme) | 25 μl |
| | 500 μl |

The digestion mixture was incubated at 37°C for 120 min. and the completion of the digestion (linearization) was confirmed by electrophoresis according to standard methods (57). The linearized DNA was extracted with phenol and chloroform and precipitated overnight with NaOAC and chilled ethanol at -20°C. The DNA was pelleted by centrifugation at 12,000 rpm, rinsed with chilled EtOH and dried.

Transfection

EL-4 mouse thymoma cells were transfected with the plasmid DNA mixture IL-2 DT #8, containing pSTneoB marker using electroporation (58).

EL-4 cells (1 x 10$^7$) were suspended in 1 ml of Hank's balanced salt solution containing 0.1% bovine serum albumin, 100 µg of IL-2 DT #8 linearized by EcoRI digestion and 1 µg of pSTneoB plasmid digested with Xhol. After incubation for 10 min. on ice, the cells were subjected to a 1500 V pulse for 20 - 50 ms (X-cell 2000, Promega Biotec), allowed to remain in the buffer for 10 min. on ice. The cells were then suspended in medium and left at room temperature for 10 min. followed by centrifugation and resuspension in RPMI 1640 medium supplemented with antibiotics (kanamycin 1 µg/ml) and 10% fetal calf serum (complete medium). The viability was determined by trypan blue exclusion and the cells distributed into 24 well trays at the density of 1x10$^5$ cells/ml/well. Selection was initiated 24 hours after transfection using 1 mg/ml G 418 in complete RPMI 1640 medium.

Similarly control neo-resistant EL-4 cells E-N were prepared by transfecting 1 µg of pSTneoB plasmid DNA digested with Xhol in the same manner described above.

Neo-resistant transfectants were isolated and tested for the presence of diphtheria toxin A (DT-A) lethal activity following induction with TPA (12-o-tetradecanoylphorbol-13 acetate). The DT-A transfected EL-4 cells are indicated as E-DT followed by the number of the clone. The pSTneo transfected cells are indicated as E-N followed by the number of the clone.

Genomic Southern Blot analysis

Genomic DNAs from E-N5 and E-DT50 cells were digested with different restriction endonucleases (EcoRI-BamHI for lanes 1 and 3; Dral for lanes 3 and 4). The resulting digests were fractionated (electrophoresed) on 1% agarose gel and transferred to nylon filters and hybridized with a $^{32}$P-labeled 0.5 Kbp Pst I-Ball fragment of DT-A according to the method of Southern (57).

As shown in Figure IV, the expected bands are 4.2 Kbp (EcoRI-BamHI) and 0.9 Kbp (Dral) respectively.

Testing of transfectants for lethal activation

It is assumed that normally, in the absence of TPA-mediated induction, the transformants E-DT are in resting condition and therefore they are not activated to transcribe the endogenous IL-2 gene (27) or the transfected chimeric gene IL-2 DT. It is important to note that the physiological induction of IL-2 can be mimicked by substances such as phorbol esters (12-o-tetradecanoylphorbol-13 acetate), also abreviated as TPA or PMA, and surface acting mitogens such as concanavalin A (Con A) data not shown. Phorbol esters mimic the mechanism of diacylglycerol activation of protein kinase C (PKC). They require the presence of phospholipids (particularly phosphatidylserine) and calcium ions.

Under resting conditions or in the absence of the transfected chimeric IL-2 DT gene the cells take up $^3$H-thymidine and metabolize it to give a certain background counts (cpm). Whereas, cells carrying the transfected IL-2 DT chimeric gene, will activate the IL-2 promoter and subsequently will transcribe and produce diphtheria toxin (DT-A) leading to cell death. As a result of DT-A production the cells cannot take up $^3$H-thymidine.

Similarly, one can monitor the lethal activation of the E-DT and control E-N transfectants using the light microscope, and the presence of cytoplasmic indicator enzymes such as lactic dehydrogenases (LDH) in the supernatants or by staining the cells following fixation.

Test Assay:

    <u>A</u>    <u>Incorporation of [$^3$H]-thymidine</u>

EL-4 cells were divided 1 : 1 one day before testing. 5 x 10$^4$ cells/well/100 µl were seeded in flat-bottom 96 well trays and incubated 60-120 min. before addition of 10 ng/ml TPA. The cells were incubated at 37°C for 24 hours under 5% $CO_2$.

About 4 hours prior to the termination of the culture, 1 µCi/well/10 µl of [$^3$H]-thymidine was added to all the wells. At the end of the incubation period the cell lysates were collected by means of an automated cell harvester onto glassfiber strips. The latter were dried and the incorporated radioactivity was measured by scintillation counting.

    <u>B</u>    <u>Cell staining</u>

For staining purposes, test cultures were set as described above except using 96-wells U bottom trays. At the end of the 24 hours incubation period, the cells were washed with Phosphate-buffered saline (PBS) and fixed with 2% paraformaldehyde. The cells were incubated at room temperature for 15 min., washed with PBS again, and stained with Giemsa's solution (Merck) at room temperature for 15 min. The trays were then gently, but extensively washed with tap water and dried, followed by the addition of 100 μl of EtOH (50%) acidified with 0.01 N HCl and the absorbance of each well was measured at 620 nm.

Results

A total of 79 E-DT transfectants were tested for TPA induced activation. As indicated earlier EL-4 transformants carrying the chimeric IL-2 DT gene will have a lethal expression leading to no or minimal incorporation of [3H]-thymidine compared to their uninduced counterparts. Any clone which showed an inhibition of 99% was selected for further testing.

Following the initial screening, 3 candidate transfectants were selected out of potential 25 clones.

As shown in Table I the neo-resistant EL-4 control cells, E-N5 and the three E-DT clones, E-DT1, E-DT49 and E-DT50 were tested with varying doses of TPA. The levels of [3H]-thymidine incorporation relative to the uninduced controls were a good reflection of the lethal expression of DT in these transfectants. In fact, even at suboptimal doses of TPA, i.e. 1 ng/ml [3H]-thymidine incorporation was inhibited by 98-99% (see Table II). It is important to note that although at concentrations of TPA 3 ng/ml [3H]-thymidine incorporation is inhibited in the E-N5 control cells, this is not a reflection of the lethal toxicity of the inducer. In fact, this was confirmed by the staining data.

Table I

| Incorporation of [3H]-thymidine | | | | |
|---|---|---|---|---|
| Transfectant clones | Control | TPA ng/ml | | |
| | | 0.3 | 1.0 | 3.0 |
| E-N5 | 567108±25793 | 550816±15621 | 411395±13859 | 87888±6859 |
| E-DT1 | 391474±15957 | 191308± 1700 | 9835± 590 | 1343± 235 |
| E-DT49 | 197676± 5823 | 19131± 252 | 756± 2 | 573± 124 |
| E-DT50 | 377870±11853 | 107390± 1431 | 3979± 542 | 3332± 566 |

Each number represents the mean cpm of triplicates ± SEM.

Table II

| Percent Inhibition of [3H]-thymidine Incorporation | | | |
|---|---|---|---|
| Transfectant clone | TPA ng/ml | | |
| | 0.3 | 1.0 | 3.0 |
| E-N5 | 3.0 | 27.0 | 84.0 |
| E-DT1 | 51.0 | 97.4 | 99.0 |
| E-DT49 | 90.0 | >99.0 | >99.0 |
| E-DT50 | 72.0 | 99.0 | 99.0 |

Inhibitory effect of Cyclosporin-A (CsA) on the incorporation of [3H]-thymidine, [3H] TdR

A    Incorporation of [3H]-Thymidine

1.5 x 10^4 EL-4 transformant cells were incubated for 30 min. with or without CsA (1 μg/ml) in 96-well flat bottom trays and the cells were stimulated with or without TPA (10 ng/ml) in the presence or absence of CsA (1 μg/ml). After incubation at 37°C under 5% $CO_2$ for 24 hours, the cells were washed with PBS, pulsed for 4 hours with 1 μCi of [3H]TdR, collected onto glassfiber strips with the aid of an automated cell harvester and the incorporated radioactivity was measured by scintillation counting.

B    Cell staining

In the case of the cell staining, cells were treated in the same way except using 96-well U bottom micro-plates. After incubation for 24 hours, cells were washed with PBS and 2% paraformaldehyde (TAAB) was added. The cells were incubated at room temperature for 15 min., washed with PBS again, and stained with Giemsa's solution (MERCK) at room temperature for 15 minutes. After washing with water, the plates were dried, 100 μl of 50% EtOH

containing 0.01 N HCl was added into each well, and the absorbance was determined at 620 nm.

Results

As described earlier, the control, E-N5 and the DT-A transfected clone, E-DET50 were used to test the potential inhibitory effect of Cyclosporine-A, a potent immunosuppressive drug utilized for the prevention of organ-transplant rejection. Incorporation of [3H]-thymidine and cell staining were used as experimental parameters to monitor the effects.

As shown in Table III, TPA-induced expression of DT, which is under the control of IL-2 promoter is a lethal event.

Table III

| Inhibition of TPA-induced lethal effect by Cyclosporin-A as measured by [3H]-thymidine incorporation | | |
|---|---|---|
| Experiment | Treatment | E-DT50 |
| I | (-) | 650400 ± 233249 |
| | 1μg/ml CsA | 661493 ± 151605 |
| | 10ng/ml TPA | 3995 ± 698 |
| | TPA+CsA | 605479 ± 83986 |
| II | (-) | 579373 ± 20031 |
| | CsA | 528245 ± 23680 |
| | TPA | 3064 ± 453 |
| | TPA+CsA | 460902 ± 23774 |

Each number represents the mean of triplications ± SEM

In both experiments I and II, more than 99% of the E-DT50 transfectants do not incorporate the radioactive label compared to the uninduced transfectants or the CsA-treated controls. However, in the presence of 1 μg/ml CsA this lethal expression of DT is prevented and the relative level of [3H]-thymidine incorporation is restored to that of the uninduced or the drug controls. These findings are consistently reproducible.

The TPA induction can also be monitored by staining the reaction trays as described above. In fact, following cell death, the cells cannot be stained and therefore the wells are clear compared to the uninduced or drug control wells. The staining parameter can be measured by directly evaluating the absorbance of the stained and fixed wells at 620 nm. As shown in Table IV, the absorbance of TPA-induced wells is dramatically reduced by 70 and 100% in experiment I and II. However, when CsA is added with the inducer TPA, the lethal activity is completely inhibited, the absorbance is restored back to the level of the uninduced or CsA-treated control wells. Again these findings are reproducible and consistent.

Table IV

| Inhibition of TPA-induced lethal effect by Cyclosporin A as measured by staining and Absorbance at 620 nm | | | |
|---|---|---|---|
| Experiment | Treatment | E-N5 | E-DT50 |
| I | (-) | 1.241 | 0.957 |
| | CsA | 0.920 | 0.957 |
| | TPA | 1.127 | 0.336 |
| | TPA+CsA | 1.106 | 1.058 |
| II | (-) | 0.975 | 1.185 |
| | CsA | 0.982 | 1.108 |
| | TPA | 1.176 | 0 |
| | TPA+CsA | 1.130 | 1.128 |

Both incorporation of [3H]-thymidine and staining measurements can reproducibly monitor the state of activation of the chimeric gene. When TPA induces the biosynthesis of DT, it leads to lethal expression, the E-DT50 transfectants are killed, thus the dramatic decrease in the incorporation of the base thymidine in the growing cells. Similarly, cell death leads to reduction or absence of staining. Cyclosporine-A can prevent the lethal outcome of TPA-induction, this is confirmed by both the [$^3$H]-thymidine incorporation and staining assays.

The experimental findings show that interference at the gene level, more specifically modulation of cellular products such as cytokines (IL-2, IL-4, IL-6, IFN etc.) at the level of transcription and translation is a very desireable objective. It allows down-regulation or inhibition of production of such cytokines, which play crucial role(s) in cell to cell interactions. The role of many cytokines, particularly IL-2 in autoimmunity, inflammation and organ transplant rejection has been well documented. The presently available immunosuppressive drugs are thought to action IL-2 and possibly other cytokine genes. Therefore, the chimeric gene of the invention, in particular the IL-2-Diph- theria toxin chimeric gene expressed in cells of lymphoid origin is a very good model for easily monitoring the effects of natural or synthetic compounds specifically modulating bioactive compound genes, e.g. IL-2 gene activation. With the use of this model, "the E-DT transfectants", it will be possible to pick up any kind of non-specific or specific inhibitor. This model can target compounds acting directly on the promoter of a bioactive compound, e.g. IL-2 gene.

Since ubiquitous and non-ubiquitous factors are involved in regulating IL-2 gene expression. It is absolutely practical to use the entire regulatory region of the IL-2 gene and express the chimeric gene construct in cells of lymphoid origin, which presumably contain a complement of the cellular regulatory factors necessary for the control of the chimeric gene expression.

This chimeric gene construct described above introduced in a lymphoid cell is novel and extremely sensitive in identifying natural or synthetic compounds with potential immunosuppressive effects.

Similar constructs incorporating instead of the promoter regulator sequence of IL-2 the promoter regulator sequence of other bioactive proteins, especially IL-4, IL-6 can be made analogously to the experimental procedure described above to provide a similar system for enabling the screening for compounds having the ability to affect the respective bioactive protein. The promoter regulator sequences for IL-4 are described in Yokota et al. (1988), Immunol. Rev., 102: 137, and for IL-6 in Akira et al., (1990), FASEB J., 4:2880.

## References

1. Cohen S. (1977), Am. J. Pathol. 88: 502

2. Mason D. W. and P. J. Morris (1986), Annu. Rev. Immunol 4: 119

3. Herberman R. B., Raynolds C. W., and J. R. Ortaldo (1986), Annu. Rev. Immunol. 4: 651

4. Kupfer A. and S. J. Singer (1989), Annu. Rev. Immunol. 7: 309

5. Rosenstreich D. and S. Mizel (1979), J. Immunol. 123: 1749

6. Farrar J. J., Benjamin W. R., Hilfiker M. L., Howard M., Farrar W. L., and J. Fuller-Farrar (1982), Immunol Rev. 63: 129

7. Taniguchi T., Matsui H., Fujita T., Hatakeyama M., Kashima N., Fuse A., and J. Hamuro (1986), Immunol. Rev. 92: 121

8. Smith K. A. (1988), Science 240: 1169

9. Howard M., Farrar J. J., Hilfiker M., Johnson B., Takatsu K., Hamaoka T. and W. E. Paul (1982), J. Exp. Med. 155 : 914

10. Mauer S. C., Hussey R. E., Cantrell D. A., Hodgdon J. C., Schlossman S. F., Smith K. A. and E. L. Reinherz (1984), Proc. Natl. Acad. Sci. U.S.A. 81: 1509

11. Klausner R. D., Samelson L. E. (1991), Cell 64: 875

12. Taniguchi T. (1988), Annu. Rev. Immunol. 6: 439

13. Henny C. S., Kuribayashi K., Kern D. E., and S. Gillis (1981), Nature, 291: 335

14. Tsudo M., Uchiyama T., Uchino H. (1984), J. Exp. Med. 160: 612

15. Rosenberg S. A., and M. T. Lotze (1986), Annu. Rev. Immunol. 4: 681

16. Okamoto Y., Minamoto S., Shimizu K., Mogami H., and T. Taniguchi (1990), Proc. Natl. Acad. Sci. U.S.A. 87: 6584

17. Leonard W. C., Depper J. M., Kanehisa M., Kroenke M., Peffer N. J., Svetlik P. B., Sullivan M. and W. C. Green (1985), Science 230: 633

18. Hatakeyama M., Tsudo M., Minamoto S., Kono T., Doi T., Miyata T., Miyasaka M., and T. Taniguchi (1989), Science 244: 551

19. Waldmann T. A. (1989), Annu. Rev. Biochem. 58: 875

20. Hatakeyama M., Kono T., Kobayashi N., Kawahara A., Levin S. D., Perlmutter R. M., and T. Taniguchi (1991), Science (in press)

21. Kroenke M., Leonard W J., Depper J. M., Arya S K., Wong-Staal F., Gallo R., Waldmann R. C, and W. C. Green (1984), Proc. Natl. Acad. Sci. U.S.A. 52: 14

22. Kroenke M., Leonard W. J., Depper J. M., and W. C. Green (1985), J. Exp. Med. 161: 1593

23. Depper J. M., Leonard W. J., Drogula C., Kroenke M., Waldmann T. A. and W. C. Green (1985), Proc. Natl.

Acad. Sci. U.S.A. 82: 4230

24. Tocci M. J., Matkovich D. A., Collier K. A., Kwok P., Dumont F., Liu S., Degudicibus S., Siekierka J. J., Chin J., and N. I. Hutchinson (1989), J. Immunol. 143: 718

25. Fujita T., Takaoka C. M., Matsui H., and T. Taniguchi (1983), Proc. Natl. Acad. Sci. U.S.A. 80: 7437

26. Fuse A., Fujita T., Yasumitsu H., Kashima N., Hasegawa K. and T. Taniguchi (1984), Nucl. Acids Res. 12:9323

27. Fujita T., Shibuya H., Ohashi T., Yamanishi K., and T. Taniguchi (1986), Cell 46: 401

28. Randak C., Brabletz T., Hergenroether M., Sobotta I., and E. Serfling (1990), EMBO J. 9: 2529

29. Brabletz T., Pietrowski I., and E. Serfling (1991), Nuc. Acids Res. 19: 61

30. Pettersson M. and W. Schaffner (1987), Genes Dev. 1:962

31. Shibuya H., and T. Taniguchi (1989), Nuc. Acids Res. 17: 9173

32. Durand D. B., Shaw J. P., Bush M. R., Replogle R. E., Belaaje R. and G. R. Crabtree (1988), Mol. Cell. Biol. 8:1715

33. Shaw J. P., Utz P., Durand D. B., Toole J. J., Emmel E. A., and G. R. Crabtree (1988), Science 241: 202

34. Nabel G. J., Gorka C., Baltimore D. (1988), Proc. Natl Acad. Sci. U.S.A. 85: 2934

35. Serfling E., Barthelmaes R., Pfeuffer I., Schenk B., Zarius S., Swoboda R., Mercurio F., and M. Karin (1989), EMBO J. 8 : 465

36. Ullman K. S., Northrop J. P., Verweij C. L. and G. R. Crabtree (1990), Annu. Rev. Immunol. 8: 421

37. Borel J., Feurer G., Gubler H., and H. Stahelin (1976), Agents Actions 6: 468

38. Showstack J., Katz P., Amend W., Bernstein L., Lipton H., O'Leary M., Bindman A. and O. Salvatierra (1989), N. Engl. J. Med. 321: 1086

39. Starzl T. E., Demetris A. J., and D. V. Thiel (1989), N. Engl. J. Med. 321: 1092

40. Kino T. H., Hatanaka M., Hashimoto M., Nishiyama T., Gotto M., Okuhara M., Kohsaka H., Aoki H. and H. Imanaka (1987), J. Antibiot. (Tokyo) 60: 1249

41. Sawada S., Suzuki C., Kawase Y. and F. Takaku (1987), J. Immunol. 139: 1797

42. Schreiber S. L. (1991), Science 283: 283

43. Elliott J. F., Lin Y., Mizel S. B., Bleackley R. C., Harnish D. G., and V. Paetkau (1984), Science 226: 1439

44. Takahashi N., Hayano T., and M. Suzuki (1989), Nature 337: 473

45. Maki N., Sekiguchi F., Nishimaki J., Miwa K., Hayano T., Takahashi N., and M. Suzuki (1990), Proc. Natl. Acad. Sci. U.S.A. 87: 5443

46. Standaert R. F., Galat A., Verdine G. L. and S. L. Schreiber (1990), Nature 346: 671

47. Handschumacher R. E., Hardnig M. W., Rice J., Drugge B. J., and D. W. Speicher (1984), Science 226: 544

48. Taniguchi T., Matsui H., Fujita T., Takaoka C., Kashima N., Yoshimoto R., and J. Hamuro (1983), Nature 302: 305

49. Yamaizumi M., Makeda E., Uchida T., and Y. Okuda (1978), Cell 15: 245

50. Pappenheimer A. M., Jr. (1977), Annu. Rev. Biochem. 46: 69

51. Leong D., Coleman K. D. and J. R. Murphy (1983), Science 220: 515

52. Greenfield L., Bjorn M. J., Horn G., Fong D., Buck G. A., Collier R. J., and D. A. Kaplan (1983), Proc. Natl. Acad. Sci. U.S.A. 80: 6853

53. Maxwell I. H., Maxwell F., and L. M. Glode (1986), Cancer Research 46: 4660

54. Maxwell F., Maxwell I. H. and L. M. Glode (1987), Mol. Cell. Bio. 7: 1576

55. Comaducci M., Ricci S., Rappuoli R. and G. Ratti (1987), Nucl. Acids Res. 15: 5897

56. Aruffo A., and B. Seed (1987), PNAS 84: 8573

57. Sambrook J., Fritsch E. F. and T. Maniatis (1989), Molecular Cloning : A laboratory manual (Cold Spring Harbor Laboratory, Cold Spring Harbor, N. Y.).

58. Doi T., Hatakeyama M., Itoh S., and T. Taniguchi (1989), EMBO J. 8: 1953

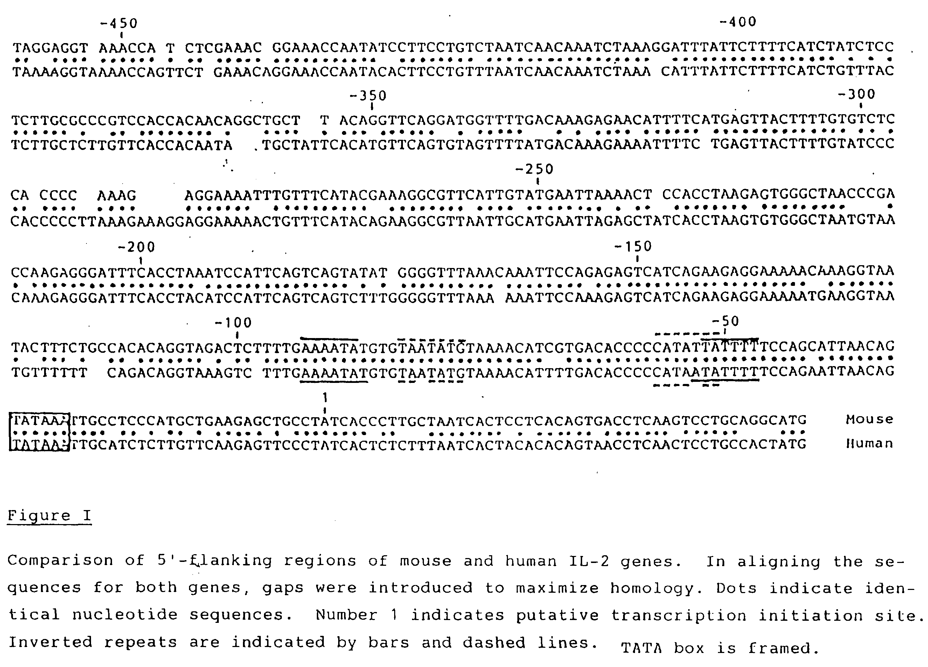

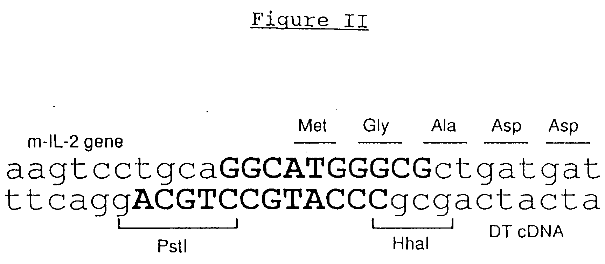

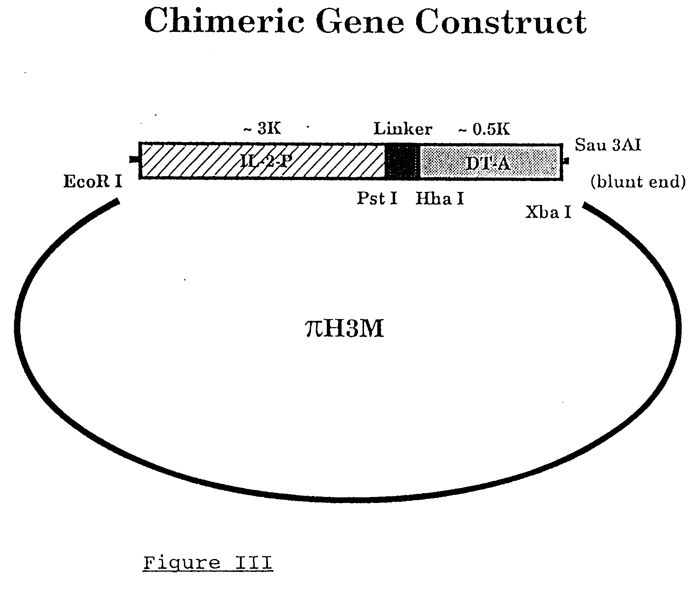

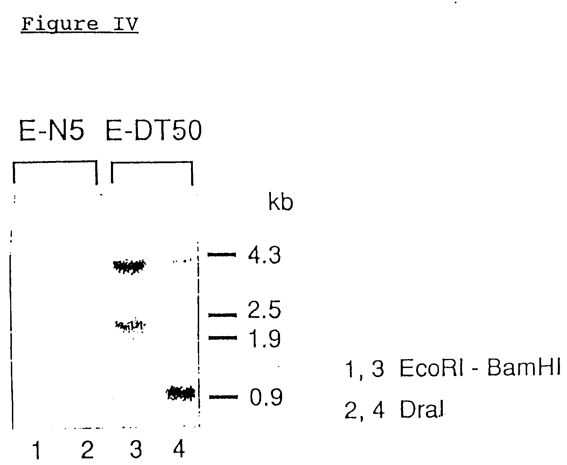

## Claims

1. A chimeric gene in which the structural gene of diphtheria toxin or diphtheria toxin A is under the control of the promoter regulator sequence of the IL-2 gene or a functional portion thereof.

2. A vector, e.g. plasmid containing a chimeric gene according to claim 1.

3. A microbial or mammalian cell transformed by a vector as defined in claim 2.

4. A mammalian cell according to claim 3.

5. A cell according to claim 4 which is a lymphoid cell.

**6.** A method of determining whether a substance is capable of affecting the regulation of expression of IL-2, which comprises subjecting a cell according to any one of claims 3 to 5 in the presence of said substance to a condition which, in the absence of said substance, is capable of inducing or suppressing the expression of said diphtheria toxin or diphtheria toxin A and observing the amount of said diphtheria toxin or diphtheria toxin A produced.

**7.** A method according to claim 6, wherein the cell is subject to a condition capable of inducing the expression of diphtheria toxin or diphtheria toxin A in the absence of said substance and the amount of said diphtheria toxin or diphtheria toxin A produced is an indication of the capability of said substance to inhibit said induction.

**8.** A method according to claim 6 or 7 wherein the production of diphtheria toxin or diphtheria toxin A is indicated by cell death.

**9.** A method according to any one of claims 6 to 8 wherein the promoter regulator sequence is stimulated by a phorbol ester, e.g. TPA or PMA, or a surface acting mitogen, e.g. concanavalin A.

**10.** The use of a cell according to any one of claims 3 to 5 in an assay method for determining the ability of a substance to regulate the expression of IL-2 by the naturally occurring gene thereof, said gene including a promoter regulator sequence.

## Patentansprüche

**1.** Chimäres Gen, bei welchem das Strukturgen des Diphtherietoxins oder des Diphtherietoxins A unter der Kontrolle der Promotor-Regulator-Sequenz des IL-2-Gens oder eines funktionellen Teiles davon steht.

**2.** Ein chimäres Gen nach Anspruch 1 enthaltender Vektor, beispielsweise ein Plasmid.

**3.** Durch einen in Anspruch 2 definierten Vektor transformierte Mikroben- oder Säugerzelle.

**4.** Säugerzelle nach Anspruch 3.

**5.** Zelle nach Anspruch 4, welche eine Lymphoidzelle ist.

**6.** Verfahren zur Feststellung, ob eine Substanz fähig ist, die Regulation der Expression von IL-2 zu beeinflussen, umfassend das Bringen einer Zelle nach einem der Ansprüche 3 bis 5, in Gegenwart der genannten Substanz, in eine Kondition, welche in Abwesenheit der genannten Substanz fähig ist, die Expression des genannten Diphtherietoxins oder Diphtherietoxins A zu induzieren oder zu unterdrücken, und die Beobachtung der produzierten Menge des genannten Diphtherietoxins oder Diphtherietoxins A.

**7.** Verfahren nach Anspruch 6, bei welchem die Zelle in eine Kondition gebracht wird, welche in Abwesenheit der genannten Substanz fähig ist, die Expression des genannten Diphtherietoxins oder Diphtherietoxins A zu induzieren, und die produzierte Menge des genannten Diphtherietoxins oder Diphtherietoxins A eine Angabe der Fähigkeit der genannten Substanz darstellt, die genannte Induktion zu unterdrücken.

**8.** Verfahren nach Anspruch 6 oder 7, bei welchem die Produktion des Diphtherietoxins oder Diphtherietoxins A durch den Zelltod angezeigt wird.

**9.** Verfahren nach einem der Ansprüche 6 bis 8, bei welchem die Promotor-Regulator-Sequenz mit einem Phorbolester, beispielsweise TPA oder PMA, oder mit einem oberflächenaktiven Mitogen, beispielsweise Concanavalin A, stimuliert wird.

**10.** Verwendung einer Zelle nach einem der Ansprüche 3 bis 5 bei einem Assay-Verfahren zur Feststellung der Fähigkeit einer Substanz zum Regulieren der Expression von IL-2 über dessen natürlich auftretendes Gen, wobei das genannte Gen eine Promotor-Regulator-Sequenz umfasst.

**Revendications**

1. Gène chimérique dans lequel le gène de structure de la toxine diphtérique ou de la toxine diphtérique A est sous le contrôle de la séquence promoteur-régulateur du gène d'IL-2 ou d'une partie fonctionnelle de celle-ci.

2. Vecteur, par exemple plasmide, contenant un gène chimérique selon la revendication 1.

3. Cellule microbienne ou de mammifère transformée par un vecteur tel que défini dans la revendication 2.

4. Cellule de mammifère selon la revendication 3.

5. Cellule selon la revendication 4 qui est une cellule lymphoïde.

6. Procédé pour déterminer si une substance est capable d'influer sur la régulation de l'expression d'IL-2 qui comprend la soumission d'une cellule selon l'une quelconque des revendications 3 à 5 en présence de ladite substance à des conditions qui, en l'absence de ladite substance, sont capables d'induire ou de supprimer l'expression de ladite toxine diphtérique ou de ladite toxine diphtérique A et l'observation de la quantité de ladite toxine diphtérique ou de ladite toxine diphtérique A produite.

7. Procédé selon la revendication 6 dans lequel la cellule est soumise à des conditions capables d'induire l'expression de la toxine diphtérique ou de la toxine diphtérique A en l'absence de ladite substance et la quantité de ladite toxine diphtérique ou de ladite toxine diphtérique A produite est une indication de la capacité de ladite substance à inhiber ladite induction.

8. Procédé selon la revendication 6 ou 7 dans lequel la production de la toxine diphtérique ou de la toxine diphtérique A est indiquée par la mort cellulaire.

9. Procédé selon l'une quelconque des revendications 6 à 8 dans lequel la séquence promoteur-régulateur est stimulée par un ester de phorbol, par exemple TPA ou PMA, ou un mitogène agissant en surface, par exemple la concanavaline A.

10. Utilisation d'une cellule selon l'une quelconque des revendications 3 à 5 dans un procédé de dosage pour déterminer l'aptitude d'une substance à réguler l'expression d'IL-2 par son gène naturel, ledit gène incluant une séquence promoteur-régulateur.

```
        -450                                                                    -400
          |                                                                       |
TAGGAGGT AAACCA T CTCGAAAC GGAAACCAATATCCTTCCTGTCTAATCAACAAATCTAAAGGATTTATTCTTTTCATCTATCTCC   .
•• ••••• •••••• • •• ••••• ••••••••••• ••••••• •••••••••••••••••   •••••••••••••••••• • • •
TAAAAGGTAAAACCAGTTCT GAAACAGGAAACCAATACACTTCCTGTTTAATCAACAAATCTAAA CATTTATTCTTTTCATCTGTTTAC

                                      -350                                                -300
                                        |                                                  |
TCTTGCGCCCGTCCACCACAACAGGCTGCT  T ACAGGTTCAGGATGGTTTTGACAAAGAGAACATTTTCATGAGTTACTTTTGTGTCTC
••••••• • •• ••••••••• •   •••• • ••• •••••• • •••• • • •••• ••••••• ••••••••••••• •• •
TCTTGCTCTTGTTCACCACAATA  .TGCTATTCACATGTTCAGTGTAGTTTTATGACAAAGAAAATTTTC TGAGTTACTTTTGTATCCC

                                                          -250
                                                            |
CA CCCC  AAAG       AGGAAAATTTGTTTCATACGAAAGGCGTTCATTGTATGAATTAAAACT CCACCTAAGAGTGGGCTAACCCGA
•• •••• •••• •       ••••••• ••••••••• ••••••••• •••• ••••••••• • •• ••••••••• •••••••••• •
CACCCCCTTAAAGAAAGGAGGAAAAACTGTTTCATACAGAAGGCGTTAATTGCATGAATTAGAGCTATCACCTAAGTGTGGGCTAATGTAA

        -200                                              -150
          |                                                 |
CCAAGAGGGATTTCACCTAAATCCATTCAGTCAGTATAT GGGGTTTAAACAAATTCCAGAGAGTCATCAGAAGAGGAAAAACAAAGGTAA
• •••••••••••••••• ••••••••••••• • • ••••••••• ••••••• •••••••••••••••• ••••••••
CAAAGAGGGATTTCACCTACATCCATTCAGTCAGTCTTTGGGGGTTTAAA AAATTCCAAAGAGTCATCAGAAGAGGAAAAATGAAGGTAA

                    -100                                        -50
                      |                                          |
                              _____                    ------|
TACTTTCTGCCACACAGGTAGACTCTTTTGAAAATATGTGTAATATGTAAAACATCGTGACACCCCCATATTATTTTTCCAGCATTAACAG
• ••• • •• ••••••• • •• •••••••••••••••••••••••••••• •••••••••••••••••••••••• •••••••
TGTTTTTT  CAGACAGGTAAAGTC TTTGAAAATATGTGTAATATGTAAAACATTTTGACACCCCCATAATATTTTTCCAGAATTAACAG
                              ¯¯¯¯¯¯¯¯        ¯ ¯¯¯¯¯                    -----  --
                        |
                        |
┌──────┐
│TATAA│TTGCCTCCCATGCTGAAGAGCTGCCTATCACCCTTGCTAATCACTCCTCACAGTGACCTCAAGTCCTGCAGGCATG   Mouse
•••••••••• •• • •• • •••••• • •••••••• •• ••••••••• • ••••••••••••• •••••• •••
│TATAA│TTGCATCTCTTGTTCAAGAGTTCCCTATCACTCTCTTTAATCACTACACACAGTAACCTCAACTCCTGCCACTATG   Human
└──────┘
```

## Figure I

Comparison of 5'-flanking regions of mouse and human IL-2 genes.  In aligning the sequences for both genes, gaps were introduced to maximize homology. Dots indicate identical nucleotide sequences.  Number 1 indicates putative transcription initiation site. Inverted repeats are indicated by bars and dashed lines.  TATA box is framed.

Figure II

m-IL-2 gene
                                    Met    Gly    Ala    Asp    Asp

aagtcctgca**GGCATGGGCG**ctgatgat
ttcagg**ACGTCCGTACCC**gcgactacta
           └─────────┘         └───────┘  DT cDNA
               PstI                 HhaI

# Chimeric Gene Construct

Figure III

# Figure IV

E-N5  E-DT50

kb

— 4.3

— 2.5
— 1.9

— 0.9

1 2 3 4

1, 3  EcoRI - BamHI

2, 4  DraI